Europäisches Patentamt

European Patent Office

Office européen des brevets

(19)

(11)  **EP 1 243 311 A1**

## (12)  EUROPEAN PATENT APPLICATION
published in accordance with Art. 158(3) EPC

(43) Date of publication:
**25.09.2002   Bulletin 2002/39**

(21) Application number: **99974179.6**

(22) Date of filing: **17.11.1999**

(51) Int Cl.[7]: **B01D 61/24**, B01D 63/02,
B01D 67/00, B01D 71/68,
A61M 1/16

(86) International application number:
**PCT/JP99/06421**

(87) International publication number:
**WO 01/036072 (25.05.2001 Gazette 2001/21)**

(84) Designated Contracting States:
**AT BE CH CY DE DK ES FI FR GB GR IE IT LI LU
MC NL PT SE**

(71) Applicant: **ASAHI MEDICAL Co., Ltd.
Tokyo 101-8482 (JP)**

(72) Inventors:
• **UEZUMI, Satoshi
Oita-shi, Oita 870-0267 (JP)**

• **YOSHIDA, Makoto
Oita-shi, Oita 870-0031 (JP)**

(74) Representative:
**von Kreisler, Alek, Dipl.-Chem. et al
Patentanwälte
von Kreisler-Selting-Werner
Postfach 10 22 41
50462 Köln (DE)**

(54)  **BLOOD TREATING MEMBRANE, BLOOD TREATING CONTAINER AND PROCESS FOR PRODUCING THE SAME**

(57)     The present inventors considered the reduction of stimuli given to blood by a blood-treating membrane, as a problem to be solved, and found that the impartment of a reducing capability to at least the surface of a blood-treating membrane surprisingly reduces the stimuli given to blood by the blood-treating membrane. Furthermore, the present inventors found a method for stably imparting reducing capability to the surface of a blood-treating membrane and the membrane surface in a blood-treating apparatus.

FIG. 1

EP 1 243 311 A1

**Description**

TECHNICAL FIELD

The present invention relates to a blood-treating membrane, a blood-treating apparatus and processes for producing them, respectively. More particularly, the present invention relates to a blood-treating membrane and a blood-treating apparatus which are used for hemodialysis, hemofiltration, plasma separation, plasma component separation, blood component adsorption, etc., and processes for producing the membrane and the apparatus, respectively.

BACKGROUND ART

In recent years, techniques such as hemocatharis therapy, blood component separation therapy and the like for treating diseases such as renal failure, hepatic diseases, autoimmune diseases, hyperlipemia, etc. have been advanced, and this advancement is good news for patients. A typical practice utilized by the techniques is a hemodialysis therapy employed for treating a patient with renal failure. Besides this therapy, hemofiltration therapy, plasmapheresis, double-filtration plasmapheresis, blood component adsorption therapy and the like begin to be generalized.

For these therapies, medical appliances using hollow fiber membranes or planar membranes are often used which are represented by hemodialyzers, plasma separators, blood component separators, etc. As to a material for a blood-treating membrane, there are well known membranes made of regenerated cellulose and membranes made of synthetic polymers such as polyacrylonitriles, polysulfones, polyethylenes, etc. As to the shape of the blood-treating membrane, there are planar membranes and hollow fiber membranes. Hollow-fiber-shaped membranes capable of having a large contact area with blood, and hence a high throughput capacity, have been the more prevalent blood-treating membranes used in recent years.

Blood-treating membranes are used as follows: a plurality of planar blood-treating membranes are generally laminated and then packed into a plastic container, or hundreds to scores of thousands of hollow fiber membranes are bundled and then packed into a cylindrical plastic container, and the planar membranes or the hollow fiber membranes are fixed by the use of a potting material to produce a semi-finished product, which is sterilized to obtain a blood-treating apparatus.

As the blood-treating apparatus, for example, a hemodialyzer is used for treating a patient with renal failure. The hemodialyzer is used in a therapy in which blood is brought into contact with a dialyzing liquid through hollow fiber or a planar membrane to move uremigenic substances and low-molecular weight proteins represented by $\beta2$ microglobulin in the blood of the patient into the dialyzing liquid, whereby the blood of the patient is depurated.

As described above, a membrane for treating blood is used in contact with blood or plasma. Therefore, the blood receives some stimulus from the blood-treating membrane which activates and produces a biologically active substance, or cells in the blood are stimulated to release a biologically active substance. As to the above phenomenon, the following, for example, has been reported: the content of oxidized ascorbic acid (dehydroascorbic acid), oxide of ascorbic acid in blood increases immediately after the start of extracorporeal circulation (Clinical Nephrology, vol. 47, 37 (1997)), and the content of oxidized phospholipids in blood collected on the outlet side of a blood-treating apparatus is higher than that in blood collected on the inlet side (Nephrology Dialysis Transplantation, vol. 10 (suppl. 3), 34 (1995)). Thus, the influences of stimuli imparted by a blood-treating membrane on a living body are vigorously investigated.

DISCLOSURE OF THE INVENTION

In view of the problems in the above prior arts, the present inventors earnestly investigated this phenomena in order to reduce stimuli given to blood by a blood-treating membrane.

Consequently, the present inventors found that by imparting a reducing capability to at least the surface of a blood-treating membrane the stimuli imparted to the blood by the membrane is surprisingly reduced. Furthermore, the present inventors found a method for imparting a stable reducing capability to the surface of a blood-treating membrane and the membrane surface in a blood-treating apparatus. Thus, the present invention has been accomplished.

That is, in order to achieve the above object, the present invention provides the following:

(1) A blood-treating membrane at least the surface of which has a reducing capability.

(2) A blood-treating membrane according to the above item (1), wherein the reducing capability is more than 100% in terms of a k value obtained by an oxidized ascorbic acid contact test (a DHAA test).

(3) A blood-treating membrane according to the above item (2), wherein said membrane comprises a polysulfone and a hydrophilic polymer.

(4) A blood-treating membrane according to the above item (1), wherein said membrane has been treated with radiation.

(5) A blood-treating membrane according to the above item (4), wherein the reducing capability is 20 or less in terms of an a* value obtained by a Neutral Red test (an NR test).

(6) A blood-treating membrane according to the above item (5), wherein said membrane comprises a polysulfone and a hydrophilic polymer.

(7) A blood-treating apparatus comprising a cylindrical container accommodating a bundle of a plurality of hollow-fiber-shaped membranes as a blood-treating membrane according to any one of the above items (1) to (6), wherein at least one end of the bundle of said membranes is fixed to the container at the end of the container so that the blood-treating apparatus has spaces inside the hollow fibers and a space outside the hollow fibers, which are separated by walls of the membranes.

(8) A process for producing a blood-treating membrane which comprises treating a membrane with radiation in water having an oxidation-reduction potential of -500 mV or less.

(9) A process for producing a blood-treating apparatus which comprises packing membranes into a container in a module, filling the module with water having an oxidation-reduction potential of -500 mV or less, and treating the membranes with radiation.

BRIEF DESCRIPTION OF THE DRAWING

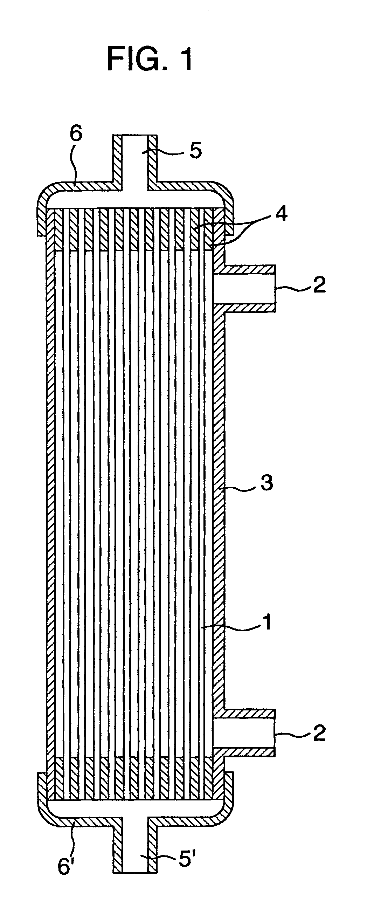

Fig. 1 is a schematic cross-sectional view showing an example of blood-treating apparatus using hollow fiber.

BEST MODE FOR CARRYING OUT THE INVENTION

The term "reducing capability" used herein means a capability to give one or more electrons to a substrate in a solution or blood (e.g. an oxidized substance in blood, among components dissolved in plasma and phospholipids constituting blood cells) to make the substrate electrically neutral or allow the substrate to assume a reduced state abundant in electrons. The degree of the reducing capability can be measured by bringing the membrane into contact with a substance having a known oxidation-reduction potential and detecting the change of the state of the substance from an oxidized state to a reduced state. In this case, the accuracy of a measured value is increased when a plurality of substances each having a known oxidation-reduction potential are used.

Previously oxidized ascorbic acid (dehydroascorbic acid) can be used as the substance having a known oxidation-reduction potential. Herein, a test using oxidized ascorbic acid is referred to as an oxidized ascorbic acid contact test (hereinafter a DHAA test). Ascorbic acid used as a substrate is present in plasma and has a known oxidation-reduction potential of 60 mV (a standard oxidation-reduction potential). Non-oxidized ascorbic acid absorbs an ultraviolet light (maximum absorption wavelength: 245 nm), and oxidized ascorbic acid absorbs no ultraviolet light. Therefore, the oxidized state and reduced state of ascorbic acid can easily be detected. The procedure for the test and the definition of a k value, a measure of the reducing capability, are described below.

Commercially available ascorbic acid is dissolved in a solution of 1 part by weight of metaphosphoric acid as a stabilizer in 49 parts by weight of pure water to a concentration of 0.002 M, and then potassium permanganate (1/8N) is added dropwise thereto to oxidise the ascorbic acid. The disappearance of an absorption at 245 nm in the ultraviolet region is confirmed by measuring the absorbance as occasion calls, and the dropwise addition of potassium permanganate is terminated at the time of substantial disappearance of the absorption to obtain a solution of oxidized ascorbic acid. Subsequently, a test substance (e.g. a blood-treating membrane) is washed several times with pure water, and 2 g of the wet membrane free from excessive water is then measured and immersed in 50 ml of said solution. Absorbance at 245 nm is measured 5 minutes and 10 minutes after the start of the immersion. A k value can be calculated by the following equation I:

$$k \, (\%) = 100 \times \text{absorbance (10 min.)/absorbance (5 min.)} \qquad \text{I}$$

As represented by equation I, the k value is the percentage of absorbance 10 minutes after the start of the contact between the test substance and the solution based on absorbance 5 minutes after the start of the contact. A k value of more than 100% means that the test substance has reduced oxidized ascorbic acid, namely, the test substance has reducing capability for oxidized ascorbic acid. A k value of 100% means that the test substance has no influence on oxidized ascorbic acid. A k value of less than 100% means that the test substance has oxidized the residual ascorbic acid.

That is, a blood-treating membrane having a k value of 100% or less neither oxidizes nor reduces substances in blood, or it oxidizes the substances. Such a blood-treating membrane is likely to affect a living body because of its oxidative stimulus. On the other hand, the blood-treating membrane of the present invention having a k value of more than 100% reduces substances in blood and lessens oxidation products to bring about effects beneficial to a living body.

The material for the blood-treating membrane of the present invention is not particularly limited. The blood-treating membrane includes, for example, regenerated-cellulose-based membranes; polysulfone-based membranes obtained by adding a hydrophilic polymer (e.g. a poly(vinylpyrrolidone), poly(vinyl alcohol) or poly(ethylene glycol)) to a polysulfone as base material in order to impart hydrophilicity to the polysulfone; cellulose triacetate membranes; poly(methyl methacrylate) membranes; polyacrylonitrile membranes; and ethylene vinyl alcohol membranes. Of these, hollow fiber membranes obtained by adding a poly(vinylpyrrolidone) to a polysulfone can be mentioned as a preferable embodiment of the present invention. Although the shape of the blood-treating membrane may be any shape such as hollow fiber, a planar membrane or the like, hollow fiber is preferable because it permits miniaturization of the blood-treating membrane.

Fig. 1 is a schematic cross-sectional view showing an example of blood-treating apparatus using hollow fibers. In Fig. 1, a bundle of two or more hollow fibers 1 is placed in a housing 3 having fluid inlets 2 and 2', and its ends are fixed to the housing 3 by the use of a potting material 4 at the ends, respectively, of the housing 3. Headers 6 and 6' having body fluid inlets 5 and 5', respectively, are attached to containers on the hollow fiber 1 open-end sides. In such a condition, the insides of the hollow fibers 1 are open to the header 6 and 6', and the outsides of the hollow fibers 1 are exposed to the inside of the housing 3. That is, the inside and outside of each hollow fiber are isolated from each other by the hollow fiber 1, the housing 3 and the potting material 4.

The blood-treating membrane should be sterilized without fail because it is brought into contact with blood. In a method for the sterilization, conventional means such as chemicals, gases, high-pressure steam, etc. can be used, though radiosterilization using γ-rays or an electron beam is preferable, for example, because it does not cause remaining of a chemical or a gas and has a high sterilizing power.

The NR test referred to herein is a test for investigating the reducing capability of the membrane and is carried out by measuring the difference of color tone of a reagent before and after the contact between the membrane and the reagent by the use of Neutral Red as an oxidation-reduction indicator to evaluate the reducing capability of the membrane.

Neutral Red permits visual and qualitative estimation of the oxidized or reduced state of the reagent because it is red in an oxidized state and colorless in a reduced state. In addition, the oxidized or reduced state of the reagent can be quantitatively estimated by expressing the color tone of the reagent in a numerical value by the use of a L\*a\*b\* color specification system according to JIS Z8729.

In the L\*a\*b\* color specification system, the red color of Neutral Red in an oxidized state is expressed in an a\* value. A high a\* value means that Neutral Red is in an oxidized state. A low a\* value means that Neutral Red is in a reduced state. The capability of the membrane to reduce Neutral Red (the reducing capability) can be determined by measuring the color tone of Neutral Red after the contact with the membrane.

Accordingly, with a decrease of the a\* value indicating the reducing capability of the membrane, the reducing capability increases and the oxidative influence of the membrane on blood decreases. The a\* value is preferably 20 or less, more preferably 15 or less. Since Neutral Red merely becomes colorless even when sufficiently oxidized, the a\* value for expressing a red color does not become less than zero and its lower limit is zero or more as a matter of course.

That is, a blood-treating membrane having an a\* value of more than 20 gives a great oxidative stimulus to blood and has remarkable influences (e.g. the release of biochemical factors) on a living body. On the other hand, the blood-treating membrane of the present invention having an a\* value of 20 or less gives surprisingly slight oxidative stimulus to blood and has little influence on blood or a living body.

The present inventors earnestly investigated a process for producing the above-mentioned blood-treating membrane, and consequently found a process comprising filling a membrane or a module with water having an oxidation-reduction potential of -500 mV or less and then treating the membrane with a radiation.

Water having a predetermined oxidation-reduction potential is produced, for example, by electrolysis of water in an electrolytic bath in which two electrodes are located with an ion-permeable diaphragm inserted between them. As an apparatus for the electrolysis, a well-known apparatus such as an apparatus for producing highly electrolyzed water can be utilized. The water thus produced is generally called highly electrolyzed alkaline water. The highly electrolyzed alkaline water obtained has a very low oxidation-reduction potential of -300 mV to -900 mV with reference to a platinum electrode and has physical characteristics very different from those of alkaline ionized water generally used as drinking water.

For example, when hollow fiber membranes are used, a bundle of hundreds to scores of thousands of hollow fiber membranes is packed into a cylindrical plastic container and fixed to the container to obtain a semi-finished product, and the semi-finished product is filled with the highly electrolyzed alkaline water produced in the manner described above. The semi-product is plugged and then the module is irradiated with a radiation such as an electron beam, γ-rays or the like to obtain a product.

A product can be obtained also by placing membranes in a container such as a test tube, filling the container with the highly electrolyzed alkaline water, treating the membranes with radiation, and then assembling the treated membranes into a module. In this case, re-sterilization is required though not limited to treatment with radiation. Therefore, it is preferable to assemble membranes into a module, fill the module with the above-mentioned highly electrolyzed alkaline water, and then sterilize the membranes with radiation.

On the other hand, because of a fear that membranes may be deteriorated by the influence of a radiation when subjected to radiosterilization, there has been proposed a process in which irradiation is carried out by the use of an antioxidant (JP-A-4-338223) and a process in which the acidification of a filling liquid is suppressed (JP-A-7-194949).

JP-A-7-194949 discloses a process in which a $\gamma$-ray treatment is carried out in a pH buffer solution or an aqueous alkali solution in order to solve the following problems of the prior art: as a result of the deterioration of membranes, the pH of water filled into a module changes remarkably to an acidic pH, resulting in difficulties at the time of use; the components of the membrane are decomposed, so that the amount of the components released by dissolution are increased; and moreover, when the membranes are stored for a long period of time, the deterioration of the membranes proceeds, so that performance characteristics of the membranes are changed.

A membrane obtained by this process, however, has no reducing capability and gives the same stimuli to blood as in the case of a conventional process, when any of the solutions mentioned as the pH buffer solution are used. The solutions are mixed aqueous solution of potassium dihydrogenphosphate and disodium hydrogenphosphate, and the solutions mentioned as the aqueous alkali solution, such as buffered aqueous solutions of an inorganic salt, an organic salt or a mixture thereof (e.g. an aqueous sodium hydrogencarbonate solution). The alkaline ionized water mentioned as the aqueous alkali solution has an oxidation-reduction potential of -100 mV to -200 mV with reference to a platinum electrode. For example, when the present inventors filled a membrane with alkaline ionized water having an oxidation-reduction potential of -180 mV, and the membrane was treated with radiation, the membrane thus treated had no reducing capability and gave the same stimuli to blood as in the case of a conventional process.

That is, a blood-treating membrane produced by the above well-known process does not inhibit the action of species with an oxidative effect produced by a treatment of radiation which remains in the membrane, though it can prevent the acidification of a filling liquid caused by the decomposition of the constituents of the membrane. When the blood-treating membrane is used in practice, there is the following possibility: the degeneration or stimulation of blood components is caused by the contact between the membrane and blood, so that biologically active substances are released, resulting in influences on a living body.

In addition, the pH of the filling liquid in a module filled with the alkaline ionized water mentioned in Example 3 in JP-A-7-194949 has a weakly acidic pH of 5.9. On the other hand, the pH of the filling liquid in a module filled with the highly electrolyzed alkaline water according to the present invention and then sterilized has an alkaline pH of 9 to 11. Therefore, the alkaline ionized water according to JP-A-7-194949 and the highly electrolyzed alkaline water according to the present invention are clearly different.

Although the reason why the object of the present invention can be achieved by treating a blood-treating membrane with radiation in the above-mentioned highly electrolyzed alkaline water is not completely clear, the following is conjectured: when the radiation treatment is carried out, radical species with a strong oxidizing power, such as hydroxyl radicals, peroxide radicals and the like are produced in water present in or around the membrane, and the above-mentioned highly electrolyzed alkaline water eliminates the radical species, or converts the radical species to radical species having a weaker oxidizing power. Therefore, it can be speculated that reducing radical species are present in the membrane but not oxidizing radical species.

Although the oxidation-reduction potential of the highly electrolyzed alkaline water to be filled into a membrane or module is preferably low, there is substantially a lower limit to the oxidation-reduction potential owing to properties of water or the capability of an apparatus for producing electrolyzed water. The lower limit is about -900 mV. Although water having an oxidation-reduction potential of -500 mV or less permits achievement of the object of the present invention, there is preferably used water having an oxidation-reduction potential of -700 mV or less, more preferably -800 mV or less.

A water-soluble antioxidant such as sodium pyrosulfite, acetone sodium bisulfite or the like may be dissolved in the above-mentioned highly electrolyzed alkaline water so long as it does not allow the oxidation-reduction potential of the highly electrolyzed alkaline water to come outside the above range. As starting water for producing the highly electrolyzed alkaline water, there can be used, for example, drinking water stipulated by waterworks law, or pure water treated with an ion-exchange membrane.

In the present invention, the radiosterilization can be carried out by the use of $\gamma$-rays, an electron beam or the like, though $\gamma$-rays are usually used from the viewpoint of, for example, the transmission of radiation by substances. In the present invention, the irradiation dose is not particularly limited though the irradiation dose of $\gamma$-rays is usually adjusted to 25 kGy or more in order to assure the sterilization.

The present invention is more concretely illustrated below with reference to examples and comparative examples, but the examples are not intended in any way to limit the scope of the invention.

The DHAA test, NR test, prostaglandin $E_2$ (hereinafter referred to as $PGE_2$) release test and granulocyte elastase release test in the examples and comparative examples were carried out according to the following methods. In all the tests, as a blood-treating membrane to be tested, there was used a membrane obtained by previously discharging a liquid filled into a module, washing the module with physiological saline, as in the case of practical use for dialysis or the like, and then disassembling the module to take out the membrane.

DHAA test

Commercially available ascorbic acid was dissolved in a solution of 1 part by weight of metaphosphoric acid as a stabilizer in 49 parts by weight of pure water to a concentration of 0.002 M, and then potassium permanganate (1/8N) was added dropwise thereto to oxidize the ascorbic acid. The disappearance of an absorption at 245 nm in the ultraviolet region was confirmed by measuring absorbance as occasion calls, and the dropwise addition of potassium permanganate was terminated when the substantial disappearance of the absorption to obtain a solution of oxidized ascorbic acid occurred. Subsequently, a test substance such as a blood-treating membrane was washed several times with pure water, and 2 g of the wet membrane free from excessive water was measured and then immersed in 50 ml of said solution. Absorbance at 245 nm was measured 5 minutes and 10 minutes after the start of the immersion and a k value was calculated by the following equation II:

$$k (\%) = 100 \times \text{absorbance (10 min.)/absorbance (5 min.)} \qquad \text{II}$$

NR test

5 Grams of the wet membrane taken out of the module and freed of excessive water was measured and then immersed for 1 hour in 50 ml of an aqueous Neutral Red solution (50 mg/L) separately prepared. Then, the membrane was taken out of the solution and freed of the remaining solution by the use of filter paper or the like, and the a* value of the membrane was measured by means of a color and color difference meter (CR-300, mfd. by MINOLTA Co., Ltd.).

$PGE_2$ test

5 Grams of the wet membrane taken out of the module was cut into small pieces and brought into contact with fresh human blood (37°C, 50 ml, heparin 5,000 IU/L) for 10 minutes, and the blood concentration of $PGE_2$ released into the blood by the stimulation of leucocytes was determined. This $PGE_2$ quantitation was carried out by the use of a $PGE_2$ EIA kit (available from FUNAKOSHI Co., Ltd.). Since $PGE_2$ was present in the blood in a certain concentration before the contact of the blood with the extraneous substance, the ratio of the difference of the $PGE_2$ concentration before and after the contact to the $PGE_2$ concentration before the contact (the percentage of increase in $PGE_2$ concentration) was calculated by the following equation III:

$$\text{Percentage of increase in PGE}_2 \text{ concentration (\%) =}$$

$$100 \times (\text{PGE}_2 \text{ concentration after contact - PGE}_2$$

$$\text{concentration before contact) / PGE}_2$$

$$\text{concentration before contact} \qquad \text{III)}$$

Granulocyte elastase release test

5 Grams of the wet membrane taken out of the module and freed of excessive water was cut into small pieces and brought into contact with fresh human blood (37°C, 50 ml, heparin 5,000 IU/L) for 10 minutes, followed by centrifugation, and the amount of granulocyte elastase released into the plasma was quantitated. For the granulocyte elastase quantitation, an EIA method was adopted. Since granulocyte elastase was present in the blood in a certain concentration before the contact of the blood with the extraneous substance, the ratio of the difference of the granulocyte elastase concentration before and after the contact to the granulocyte elastase concentration before the contact (the percentage of increase in GE concentration) was calculated by the following equation IV:

Percentage of increase in GE concentration (%) =

100 × (GE concentration after contact - GE

concentration before contact) / GE

concentration before contact                                                                IV

Example 1

Hollow fiber membranes made of polysulfone and a poly(vinylpyrrolidone) were assembled into a module having an effective membrane area of 1.5 m$^2$. The module was filled with highly electrolyzed alkaline water with an oxidation-reduction potential of -820 mV produced by means of an apparatus for producing highly electrolyzed alkaline water (SL-2500, mfd. by TOA Medical Electronics Co., Ltd.), and was treated with high-pressure steam (121°C, 20 minutes). The liquid filled into the module was previously discharged, and the module was washed with physiological saline as in the case of the practical use for dialysis or the like. Then, the resulting blood-treating membranes were taken out by disassembling the module, and these membranes were subjected to the DHAA test, NR test, PGE$_2$ release test and granulocyte elastase release test. The results are shown in Table 1.

Example 2

Hollow fiber membranes made of a polysulfone and a poly(vinylpyrrolidone) were assembled into a module having an effective membrane area of 1.5 m$^2$. The module was filled with highly electrolyzed alkaline water with an oxidation-reduction potential of -820 mV produced by means of an apparatus for producing highly electrolyzed alkaline water (SL-2500, mfd. by TOA Medical Electronics Co., Ltd.) before irradiating the module with a radiation. Then, the module was sterilized by irradiation with 25 kGy of γ-rays. The liquid filled into the module was previously discharged, and the module was washed with physiological saline as in the case of the practical use for dialysis or the like. Thereafter, the resulting blood-treating membranes were taken out by disassembling the module, and these membranes were subjected to the DHAA test, NR test, PGE$_2$ release test and granulocyte elastase release test. The results are shown in Table 1.

Example 3

The process of Example 2 was repeated except for filling with highly electrolyzed alkaline water having an oxidation-reduction potential of -740 mV. Then, the DHAA test, NR test, PGE$_2$ release test and granulocyte elastase release test were carried out. The results are shown in Table 1.

Example 4

The process of Example 2 was repeated except for the step of filling a module with highly electrolyzed alkaline water having an oxidation-reduction potential of -510 mV. Then, the DHAA test, NR test, PGE$_2$ release test and granulocyte elastase release test were carried out. The results are shown in Table 1.

Comparative Example 1

Hollow fiber membranes made of a polysulfone and a poly(vinylpyrrolidone) were assembled into a module having an effective membrane area of 1.5 m$^2$. The module was filled with pure water and treated with high-pressure steam (121°C, 20 minutes). The liquid filled into the module was previously discharged, and the module was washed with physiological saline as in the case of the practical use for dialysis or the like. Then, the resulting blood-treating membranes were taken out by disassembling the module, and these membranes were subjected to the DHAA test, NR test, PGE$_2$ release test and granulocyte elastase release test. The results are shown in Table 1.

Comparative Example 2

The process of Example 2 was repeated except for the step of filling a module with highly electrolyzed alkaline water having an oxidation-reduction potential of -450 mV. Then, the DHAA test, NR test, PGE$_2$ release test and granulocyte elastase release test were carried out. The results are shown in Table 1.

Comparative Example 3

The process of Example 2 was repeated except for the step of filling a module with alkaline ionized water having an oxidation-reduction potential of -180 mV. Then, the DHAA test, NR test, $PGE_2$ release test and granulocyte elastase release test were carried out. The results are shown in Table 1.

Comparative Example 4

A module was produced in the same manner as in Example 2 except for using a liquid with an oxidation-reduction potential of +98 mV prepared by dissolving sodium hydrogencarbonate (0.1 w/v%) in pure water treated with an ion-exchange membrane, in place of the filling liquid used in Example 2. The module was irradiated with 25 kGy of $\gamma$-rays and then subjected to the same washing operation as in Example 2. Thereafter, the resulting membranes were taken out of the module and subjected to the DHAA test, NR test, $PGE_2$ release test and granulocyte elastase release test. The results are shown in Table 1.

Comparative Example 5

The process of Comparative Example 4 was repeated except for the step of filling a module with a liquid with an oxidation-reduction potential of +146 mV, prepared in the same manner as in Comparative Example 4, except for using sodium hydroxide (0.1 w/v%) in place of sodium hydrogencarbonate. After the module irradiated with 25 kGy of $\gamma$-rays was subjected to the same washing operation as in Example 2, the resulting membranes were taken out of the module and subjected to the DHAA test, NR test, $PGE_2$ release test and granulocyte elastase release test. The results are shown in Table 1.

Comparative Example 6

A module was produced in the same manner as in Example 2 except for using pure water treated with an ion-exchange membrane, in place of the filling liquid used in Example 2. The module was irradiated with 25 kGy of $\gamma$-rays and then subjected to the same washing operation as in Example 2. Thereafter, the resulting membranes were taken out of the module and subjected to the DHAA test, NR test, $PGE_2$ release test and granulocyte elastase release test. The results are shown in Table 1.

INDUSTRIAL APPLICABILITY

According to the present invention, a blood-treating membrane and a blood-treating apparatus which give surprisingly reduced stimuli to blood are provided by imparting a reducing capability to at least the surface of a blood-treating membrane. There is also provided a method for stably imparting a reducing capability to the surface of a blood-treating membrane and the membrane surface in a blood-treating apparatus.

Table 1

| | Filling liquid | Oxidation-reduction potential (mV) | Sterilization method | k value (%) | a* value | Percentage of increase in $PGE_2$ concentration (%) | Percentage of increase in GE concentration (%) |
|---|---|---|---|---|---|---|---|
| Example 1 | Highly electrolyzed alkaline water | −820 | High-pressure steam | 104 | 12 | 21 | 22 |
| Example 2 | Highly electrolyzed alkaline water | −820 | γ-rays | 104 | 14 | 20 | 19 |
| Example 3 | Highly electrolyzed alkaline water | −740 | γ-rays | 103 | 16 | 22 | 45 |
| Example 4 | Highly electrolyzed alkaline water | −510 | γ-rays | 103 | 18 | 28 | 51 |
| Comparative Example 1 | Pure water | +182 | High-pressure steam | 97 | 13 | 73 | 128 |
| Comparative Example 2 | Highly electrolyzed alkaline water | −450 | γ-rays | 98 | 29 | 89 | 147 |
| Comparative Example 3 | Alkaline ionized water | −180 | γ-rays | 95 | 28 | 87 | 204 |
| Comparative Example 4 | Sodium hydrogencarbonate solution | +98 | γ-rays | 94 | 27 | 96 | 135 |
| Comparative Example 5 | Sodium hydroxide solution | +146 | γ-rays | 91 | 28 | 101 | 160 |
| Comparative Example 6 | Pure water | +180 | γ-rays | 92 | 31 | 98 | 298 |

Sterilization method:　　High-pressure steam sterilization : 121°C, 20 min.

γ-ray sterilization　　　　　　　　: 25 kGy

**Claims**

1. A blood-treating membrane at least the surface of which has a reducing capability.

2. The blood-treating membrane according to claim 1, wherein the reducing capability is more than 100% in terms of a k value obtained by an oxidized ascorbic acid contact test (a DHAA test).

3. The blood-treating membrane according to claim 2, wherein said membrane comprises a polysulfone and a hydrophilic polymer.

4. The blood-treating membrane according to claim 1, wherein said membrane has been treated with radiation.

5. The blood-treating membrane according to claim 4, wherein the reducing capability is 20 or less in terms of an a* value obtained by a Neutral Red test (an NR test).

6. The blood-treating membrane according to claim 5, wherein said membrane comprises a polysulfone and a hydrophilic polymer.

7. A blood-treating apparatus comprising a cylindrical container accommodating a bundle of a plurality of hollow-fiber-shaped membranes as a blood-treating membrane according to any one of claims 1 to 6, wherein at least one end of the bundle of said membranes is fixed to the container at the end of the container so that the blood-treating apparatus has spaces inside the hollow fibers and a space outside the hollow fibers, which are separated by walls of the membranes.

8. A process for producing a blood-treating membrane which comprises treating a membrane with radiation in water having an oxidation-reduction potential of -500 mV or less.

9. A process for producing a blood-treating apparatus which comprises packing membranes into a container in a module, filling the module with water having an oxidation-reduction potential of -500 mV or less, and treating the membranes with radiation.

# FIG. 1

## INTERNATIONAL SEARCH REPORT

| | International application No. |
|---|---|
| | PCT/JP99/06421 |

A. CLASSIFICATION OF SUBJECT MATTER
    Int.Cl⁷  B01D61/24, B01D63/02, B01D67/00, B01D71/68, A61M1/16

According to International Patent Classification (IPC) or to both national classification and IPC

B. FIELDS SEARCHED

Minimum documentation searched (classification system followed by classification symbols)
    Int.Cl⁷  B01D61/24, B01D63/02, B01D67/00, B01D71/68, A61M1/16

Documentation searched other than minimum documentation to the extent that such documents are included in the fields searched
    Jitsuyo Shinan Koho          1922-1996    Toroku Jitsuyo Shinan Koho  1994-1999
    Kokai Jitsuyo Shinan Koho    1971-1999    Jitsuyo Shinan Toroku Koho  1996-1999

Electronic data base consulted during the international search (name of data base and, where practicable, search terms used)
    WPI(DIALOG)

C. DOCUMENTS CONSIDERED TO BE RELEVANT

| Category* | Citation of document, with indication, where appropriate, of the relevant passages | Relevant to claim No. |
|---|---|---|
| EX | JP, 2000-24104, A (Asahi Medical Co., Ltd.), 25 January, 2000 (25.01.00), Claims; Column 3, line 21 to Column 4, line 7 (Family: none) | 1-9 |
| X | EP, 194483, A (AIR PRODUCTS AND CHEMICALS, INC.), 17 September, 1986 (17.09.86), Claim 6 & US, 4617029, A    & JP, 6-204019, A, Claim 6 | 1 |
| A | JP, 7-194949, A (KAWASUMI LABORATORIES, INC.), 01 August, 1995 (01.08.95), Claims    (Family: none) | 1-9 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 03 February, 2000 (03.02.00) | 15 February, 2000 (15.02.00) |

| Name and mailing address of the ISA/ | Authorized officer |
|---|---|
| Japanese Patent Office | |
| Facsimile No. | Telephone No. |

Form PCT/ISA/210 (second sheet) (July 1992)